# EUROPEAN PATENT APPLICATION

(11) **EP 4 710 788 A1**
(43) Date of publication of application: **18.03.2026**
(21) Application number: 24806524.5
(22) Date of filing: 11.05.2024
(51) Int. Cl.: A24F 40/42, A24F 40/20

(54) **AEROSOL GENERATING SYSTEM AND AEROSOL GENERATING SUBSTRATE ASSEMBLY**

(30) Priority: 12.05.2023 CN 202310539307
(71) Applicant: Smoore International Holdings Limited, Grand Cayman, KY1-1111 (KY)
(72) Inventor: LUO, Yongjie, Shenzhen, Guangdong 518102 (CN); ZHENG, Wei, Shenzhen, Guangdong 518102 (CN); XIONG, Wei, Shenzhen, Guangdong 518102 (CN)
(74) Representative: Winter, Brandl - Partnerschaft mbB
(86) International application number: PCT/CN2024/092724
(87) International publication number: WO 2024/235172

(57) **Abstract**

The present disclosure relates to an aerosol generating system and an aerosol generating substrate assembly. The aerosol generating substrate assembly includes a banded aerosol generating substrate arranged movably and an atomization cavity for allowing the aerosol generating substrate to be heated and atomized to form an aerosol, where the atomization cavity is provided with an air outlet channel, the air outlet channel has an axial direction, and a set included angle is formed between the axial direction of the air outlet channel and a movement direction of the aerosol generating substrate in the atomization cavity; and the set included angle is greater than zero degree and less than one hundred and eighty degrees. By forming the set included angle greater than zero degree and less than one hundred and eighty degrees between the axial direction of the air outlet channel and the movement direction of the aerosol generating substrate in the atomization cavity, aerosol output efficiency can be improved, and an aerosol output effect can be ensured, thereby improving user experience.

## Description

### TECHNICAL FIELD

The present disclosure relates to the field of atomization, and in particular to an aerosol generating system and an aerosol generating substrate assembly.

### BACKGROUND

In the related technologies, an aerosol generating system is capable of serving to heat a cylindrical aerosol generating substrate to generate an aerosol. Atomization media before and after the aerosol generating substrate is atomized are blended together, which easily blends smell of the aerosol, and affects inhaling experience of a user. In some related technologies, the aerosol generating substrate can be made into a band shape to solve the above problem. However, in the related technologies, the banded aerosol generating substrate typically has the defects of slow gas outlet and a poor gas outlet effect after being atomized in an atomization cavity.

### SUMMARY

The technical problem to be solved by the present disclosure is to provide an improved aerosol generating system and aerosol generating substrate assembly.

The technical solution used in the present disclosure to solve the technical problem of the present disclosure is as follows: an aerosol generating substrate assembly is constructed and includes a banded aerosol generating substrate arranged movably and an atomization cavity for allowing the aerosol generating substrate to be heated and atomized to form an aerosol, where
the atomization cavity is provided with an air outlet channel, the air outlet channel has an axial direction, and a set included angle is formed between the axial direction of the air outlet channel and a movement direction of the aerosol generating substrate in the atomization cavity; and the set included angle is greater than zero degree and less than one hundred and eighty degrees.

In some embodiments, a heating region is arranged in the atomization cavity, and the aerosol generating substrate is movably arranged in the heating region in a penetrating manner; and
the set included angle is formed between the axial direction of the air outlet channel and a penetration direction in which the aerosol generating substrate is arranged in the heating region in a penetrating manner.

In some embodiments, the heating region has a start point and an end point in the direction in which the aerosol generating substrate is arranged in a penetrating manner; and the set included angle is formed between connecting lines between a central axis of the air outlet channel and the start point and between the central axis of the air outlet channel and the end point.

In some embodiments, the atomization cavity has an inlet and an outlet; the inlet is in communication with the atomization cavity for the aerosol generating substrate to be heated to enter the atomization cavity; and the outlet is in communication with the atomization cavity for the heated aerosol generating substrate to output.

In some embodiments, the inlet and the outlet are provided on two different sides of the air outlet channel; or
the inlet and the outlet are provided on the same side of the air outlet channel.

In some embodiments, the height of the outlet is greater than or equal to the height of the inlet; and
the height of the inlet is greater than or equal to the thickness of the aerosol generating substrate.

In some embodiments, the heights/height of the inlet and/or the outlet range/ranges from 0.8 mm to 2.5 mm; and/or
the thickness of the aerosol generating substrate ranges from 0.15 mm to 0.35 mm.

In some embodiments, the widths/width of the inlet and/or the outlet are/is greater than the width of the aerosol generating substrate.

In some embodiments, the ratios/ratio of the heights of the inlet and/or the outlet to the height of the atomization cavity are/is 0.12 to 0.40.

In some embodiments, the width of the atomization cavity is greater than or equal to the width of the heating region; and
the width of the heating region is greater than the width of the aerosol generating substrate.

An aerosol generating system is further constructed in the present disclosure. The aerosol generating system includes an aerosol generating device and the aerosol generating substrate assembly of the present disclosure.

The aerosol generating system and the aerosol generating substrate assembly of the present disclosure are implemented, and have the following beneficial effects: by forming the set included angle greater than zero degree and less than one hundred and eighty degrees between the axial direction of the air outlet channel and the movement direction of the aerosol generating substrate in the atomization cavity, aerosol output efficiency can be improved, and an aerosol output effect can be ensured, thereby improving user experience.

### BRIEF DESCRIPTION OF THE DRAWINGS

The present disclosure will be further described below in combination with the accompanying drawings and the embodiments. In the figures,
FIG. 1 is a schematic structural diagram of an aerosol generating system in Embodiment 1 of the present disclosure;
FIG. 2 is a cutaway view of the aerosol generating system shown in FIG. 1;
FIG. 3 is a schematic exploded view of a local structure of the aerosol generating system shown in FIG. 1;
FIG. 4 is a schematic structural diagram of an aerosol generating substrate assembly in the aerosol generating system shown in FIG. 3;
FIG. 5 is a schematic exploded view of a local structure of the aerosol generating substrate assembly shown in FIG. 4;
FIG. 6 is a schematic diagram of the local structure of the aerosol generating substrate assembly shown in FIG. 5;
FIG. 7 is a schematic structural diagram of an aerosol generating substrate in the aerosol generating substrate assembly shown in FIG. 6;
FIG. 8 is a schematic structural diagram of an atomization housing in the aerosol generating substrate assembly shown in FIG. 6;
FIG. 9 is a schematic structural diagram of an aerosol generating device in the aerosol generating system shown in FIG. 3;
FIG. 10 is a schematic exploded view of a local structure of the aerosol generating device in the aerosol generating system shown in FIG. 9;
FIG. 11 is a schematic diagram of a local structure of an aerosol generating substrate assembly of an aerosol generating system in Embodiment 2 of the present disclosure;
FIG. 12 is a schematic enlarged view of a local structure of the aerosol generating substrate assembly shown in FIG. 11; and
FIG. 13 is a cutaway view of a local structure of the aerosol generating substrate assembly shown in FIG. 11.

Description of reference numerals: 100, aerosol generating system; 10, aerosol generating substrate assembly; 11, box body; 11a, first box body; 11b, second box body; 110, cavity; 110a, storage space; 110b, accommodating space, 111, air intake vent; 112, accommodating portion; 12, aerosol generating substrate; 121, base band; 121a, first surface; 121b, second surface; 122, medium layer; 13, first accommodating structure; 131, storage disk; 1311, first disk body; 1312, second disk body; 14, second accommodating structure; 141, accommodating disk; 1411, first disk body; 1412, second disk body; 15, atomization housing; 15a, body portion; 15b, extension portion; 151, atomization cavity; 1510, heating region; 152, inlet, 153, outlet; 16, guide structure; 161, first guide roller; 162, second guide roller; 163, third guide roller; 164, fourth guide roller; 17, blocking structure; 171, first blocking structure; 172, second blocking structure; 20, aerosol generating device; 21, machine body; 211, enclosure; 211a, first housing; 211b, second housing; 2111, accommodating cavity; 2112, assembly port; 212, cover body; 22, heating structure; 221, base; 222, heating element; 23, driving assembly; 24, sealing structure; 25, power supply; 26, main control board; and 30, mouthpiece assembly.

### DETAILED DESCRIPTION

In order to understand the technical features, objective, and effects of the present disclosure more clearly, specific implementations of the present disclosure are now described in detail with reference to the accompanying drawings. In the following description, it should be understood that the terms "upper", "longitudinal", "transverse", "inner", "outer", "axial", "radial", etc. indicate azimuthal or positional relationships based on those shown in the accompanying drawings and are constructed and operative in a particular orientation only for ease of description of the technical solution, are not intended to indicate that the device or element indicated needs to have a particular orientation, and thus cannot be construed as a limitation on the present disclosure.

It should be further noted that unless explicitly specified and defined otherwise, the terms "mount", "connect", "connected", "fix", "arrange", etc. should be understood in a broad sense. For instance, they can denote fixed connection, detachable connection, or integral connection, denote mechanical connection, or electric connection, denote direct connection or indirect connection through an intermediate medium, or denote communication between interiors of two elements or interaction between two elements. When an element is referred to as being "above" or "below" another element, the element can be "directly" or "indirectly" located above the another element, or one or more intervening elements may exist. The terms "first", "second", "third", etc. are only for the convenience of describing the technical solution, and cannot be understood as indicating or implying relative importance or implicitly specifying the number of technical features indicated. Thus, the features limited by "first", "second", "third", etc. can explicitly or implicitly include one or more of the features. Those of ordinary skill in the art can understand the specific meanings of the above terms in the present disclosure according to specific situations.

In the following description, for the purpose of description rather than limitation, specific details such as the specific system structure and technology are provided to thoroughly understand the embodiments of the present disclosure. However, those skilled in the art should be aware that the present disclosure may alternatively be implemented in other embodiments without these specific details. In other cases, detailed descriptions of well-known systems, devices, circuits, and methods are omitted to prevent unnecessary details from hindering the description of the present disclosure.

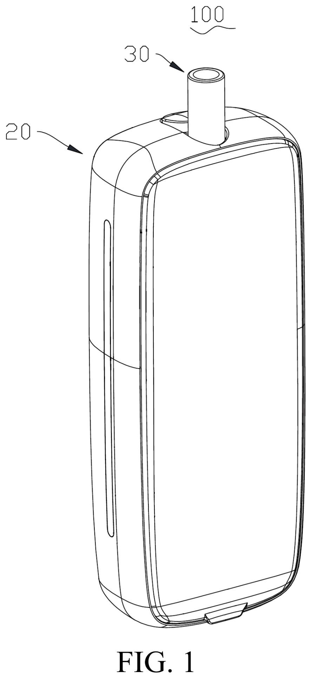

FIG. 1 shows Embodiment 1 of an aerosol generating system of the present disclosure. The aerosol generating system 100 may generate an aerosol for a user to inhale. The aerosol generated by the aerosol generating system 100 has the advantages of excellent taste and excellent user experience.

The aerosol generating system 100 includes an aerosol generating substrate assembly 10 and an aerosol generating device 20. The aerosol generating substrate assembly 10 may be mounted on the aerosol generating device 20, and is used for generating the aerosol when heated. The aerosol generating substrate assembly 10 may be connected to the aerosol generating device 20. The connection may be detachable connection, thereby replacing the aerosol generating substrate assembly 10 conveniently. In some other embodiments, connection may be fixed and integral connection. The aerosol generating device 20 may generate and output the aerosol by heating an aerosol generating substrate 12 in the aerosol generating substrate assembly 10 to atomize the aerosol generating substrate.

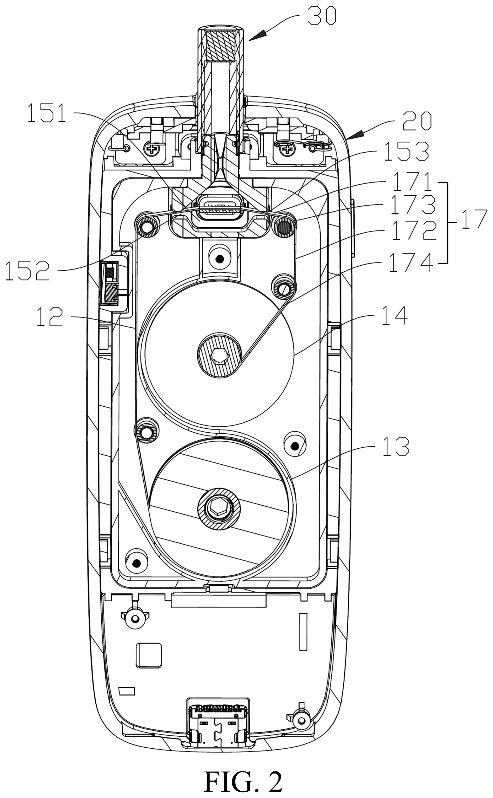

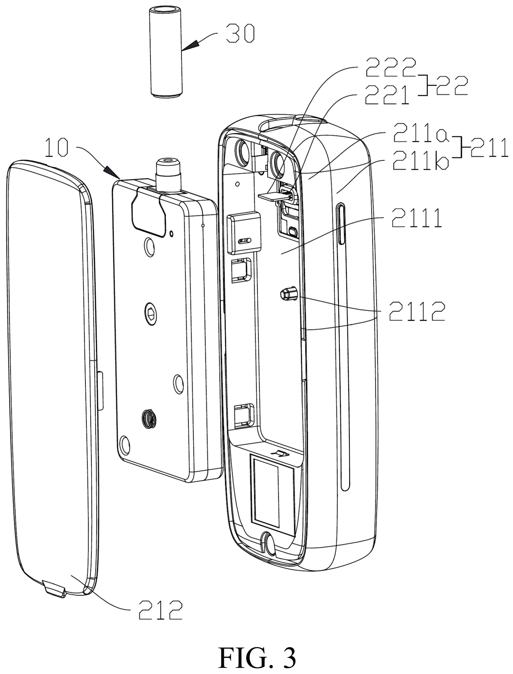

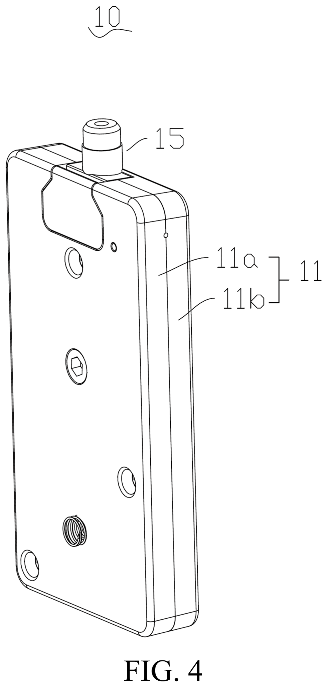

As shown in FIG. 2 to FIG. 4, in the embodiment, the aerosol generating substrate assembly 10 includes a box body 11 and an aerosol generating substrate 12. The box body 11 is used for accommodating the aerosol generating substrate 12. The aerosol generating substrate 12 may be atomized to generate an aerosol in a heated state. Understandably, in some other embodiments, a box body 11 may be omitted. An aerosol generating substrate 12 may be directly mounted in an aerosol generating device 20.

In the embodiment, the box body 11 may be of a transparent structure, and may be a transparent plastic box, for example. Clearly, understandably, in some other embodiments, a box body 11 is not limited to a transparent structure, and may be a non-transparent structure, for example, a non-transparent plastic box or a non-transparent metal box. In some embodiments, a box body 11 may include a first box body 11a and a second box body 11b. Shapes and sizes of the first box body 11a and the second box body 11b are equivalent. In some embodiments, a first box body 11a and a second box body 11b are approximately cuboid-shaped. The first box body 11a and the second box body 11b may not be limited to being cuboid-shaped, for example, cylindrically cube-shaped or irregular. A cavity 110 is formed on an inner side of each of the first box body 11a and the second box body 11b, and the first box body and the second box body are open structures. One side of the first box body 11a having a splicing port may be spliced to one side of the second box body 11b having a splicing port. The first box body 11a and the second box body 11b may be connected and fixed by arranging a connecting structure. The connecting structure may be a screwing assembly, a clamping assembly, a hinge structure, or any other type. In some embodiments, a connecting structure may alternatively be omitted. A first box body 11a and a second box body 11b may be connected by using a conventional ultrasonic technology.

In the embodiment, the aerosol generating substrate assembly 10 further includes air intake vents 111. The air intake vents 111 may be provided on the box body 11, are in communication with the cavities 110, and may allow an external gas to enter the cavities 110 to further enter an atomization cavity 151. Specifically, the two air intake vents 111 are provided, and the two air intake vents 111 may be provided on two opposite sides of the box body 11. The air intake vents 111 may be circular through holes, and semi-circular through holes may be provided on the first box body 11a and the second box body 11b respectively. When the first box body 11a and the second box body 11b are spliced, the circular air intake vents 111 may be formed by splicing the semi-circular through holes on the first box body 11a and the second box body 11b. Understandably, in some other embodiments, air intake vents 111 are not limited to being provided on a first box body 11a and a second box body 11b, and may be provided only on the first box body 11a or provided only on the second box body 11b. In some other embodiments, air intake vents 111 are not limited to being circular, and may be square or other shapes.

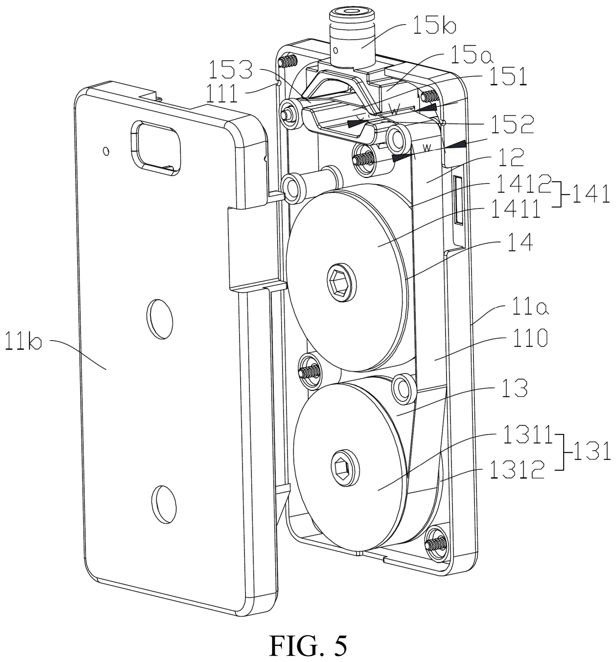

As shown in FIG. 5 to FIG. 7, in the embodiment, the aerosol generating substrate 12 is banded in a whole, and may be movably wound. In some embodiments, the thickness T of an aerosol generating substrate 12 may range from 0.15 mm to 0.35 mm (values at two ends are included). In the embodiment, the thickness T of the aerosol generating substrate 12 may be selected as 0.25 mm. In the embodiment, the aerosol generating substrate 12 includes a base band 121 and a medium layer 122. The base band 121 is used for carrying the medium layer 122. The medium layer 122 is formed on the base band 121. Specifically, the medium layer 122 may be formed by applying an atomization medium to the base band 121. The atomization medium may be a liquid and curable atomization medium, or may be a pasty atomization medium or a solid atomization medium. In some other embodiments, a medium layer 122 is not limited to being formed by applying an atomization medium, and may alternatively be formed by pressing or pasting the atomization medium on a base band 121, for example.

In the embodiment, the base band 121 is longitudinally arranged, and has a first surface 121a and a second surface 121b arranged opposite to each other. The first surface 121a and the second surface 121b may be surfaces defined by a long edge and a wide edge of the base band 121. The first surface 121a may carry the medium layer 122. In some embodiments, a base band 121 may be an easily heat-conducting metal sheet, for example, an aluminum foil or a copper foil. Certainly, in some other embodiments, a base band 121 is not limited to a metal sheet, and may alternatively be a metal mesh. The metal mesh may be formed by braiding a metal wire, or may be formed by providing a plurality of through holes on the metal sheet.

Specifically, the medium layer 122 may be formed on the entire first surface 121a of the base band 121, and may be formed by uniformly applying the atomization medium in a length direction of the base band 121. In some embodiments, the thickness of a medium layer 122 at any position of the base band 121 may be the same. Clearly, understandably, in some other embodiments, the thicknesses of at least two positions of a medium layer 122 on a base band 121 may be different. In some other embodiments, an atomization medium may be applied to a first surface 121a of a base band 121 in a segmented manner to form a plurality of segments of medium layers 122. The plurality of segments of medium layers 122 may be equidistantly distributed, and may extend by the same length in the length direction of the base band 121. Understandably, in some other embodiments, at least two segments of medium layers 122 extend by different lengths in a length direction of a base band 121.

In the embodiment, the aerosol generating substrate assembly 10 further includes a first accommodating structure 13. The first accommodating structure may be used for accommodating the aerosol generating substrate 12 to be heated. The first accommodating structure 13 may be mounted between the first box body 11a and the second box body 11b, and is connected to the first box body 11a and the second box body 11b. In some other embodiments, a first accommodating structure 13 is not limited to being mounted in a box body 11, and may alternatively be directly mounted in the aerosol generating device 20.

In the embodiment, the first accommodating structure 13 may be a storage disk 131 for storing the aerosol generating substrate 12 to be heated. The aerosol generating substrate 12 to be heated may be wound around the storage disk 131, and the storage disk 131 may drive the aerosol generating substrate 12 to be heated to be conveyed through rotation. In some embodiments, a storage disk 131 includes a first disk body 1311 and a second disk body 1312. The first disk body 1311 and the second disk body 1312 may be approximately circular, and the radial dimensions of the first disk body 1311 and the second disk body 1312 may be approximately equivalent. The first disk body 1311 is connected to the second disk body 1312 through a hollow winding drum. The winding drum may be formed at an axis of the first disk body 1311 and/or the second disk body 1312, or may be arranged separately, and two ends of the winding drum are connected to the first disk body 1311 and the second disk body 1312 respectively. The radial dimension of the winding drum is less than the radial dimensions of the first disk body 1311 and the second disk body 1312, and the aerosol generating substrate 12 to be heated may be wound around the winding drum. Understandably, in some other embodiments, a first disk body 1311 and a second disk body 1312 may not be limited to being circular, and may alternatively be square. The first disk body 1311 and the second disk body 1312 may be used for blocking the aerosol generating substrate 12 from being separated in a winding process. In some embodiments, a first disk body 1311 and a second disk body 1312 may alternatively be omitted. In some embodiments, a storage disk 131 is rotatably connected to a first box body 11a and/or a second box body 11b, and may drive an aerosol generating substrate 12 to be heated to be wound and unwound through rotation, thereby accommodating and conveying the aerosol generating substrate 12 to be heated.

In the embodiment, the aerosol generating substrate assembly 10 further includes a second accommodating structure 14. The second accommodating structure may be used for accommodating a base band 121 of the atomized aerosol generating substrate 12. That is, the second accommodating structure may accommodate the base band 121 from which the medium layer 122 is separated. In some embodiments, an accommodating disk 141 is arranged between a first box body 11a and a second box body 11b, and is connected to the first box body 11a and the second box body 11b. In some other embodiments, a second accommodating structure 14 is not limited to being mounted in a box body 11, and may alternatively be directly mounted in an aerosol generating device 20.

In the embodiment, the second accommodating structure 14 may include an accommodating disk 141 for accommodating the heated aerosol generating substrate 12. The heated aerosol generating substrate 12 may be wound around the accommodating disk 141. In some embodiments, an accommodating disk 141 includes a third disk body 1411 and a fourth disk body 1412. The third disk body 1411 and the fourth disk body 1412 are circular, and the radial dimensions of the third disk body and the fourth disk body are approximately equivalent. The third disk body 1411 and the fourth disk body 1412 are arranged at an interval and are connected through a hollow reel. The reel may be formed at an axis of the third disk body 1411 and/or the fourth disk body 1412, or may be arranged separately, and two ends of the reel are connected to the third disk body 1411 and the fourth disk body 1412 respectively. The reel may be cylindrical, the radial dimension of the reel is less than the radial dimensions of the third disk body 1411 and the fourth disk body 1412, and the heated base band 121 may be wound around the reel. In some embodiments, a third disk body 1411 and a fourth disk body 1412 may not be limited to being circular, and may alternatively be square. The third disk body 1411 and the fourth disk body 1412 may block a base band 121 from being separated. In some embodiments, a third disk body 1411 and a fourth disk body 1412 may be omitted. In some embodiments, an accommodating disk 141 may be rotatably connected to a first box body 11a and/or a second box body 11b, and may drive an aerosol generating substrate 12 to be wound around the accommodating disk through rotation.

In the embodiment, the accommodating disk 141 may include a driving wheel, and the storage disk 131 may include a driven wheel. The driving wheel moves to drive the driven wheel to move. Because the aerosol generating substrate 12 is wound around the storage disk 131 and the accommodating disk 141, the storage disk 131 may rotate under a driving effect of the accommodating disk 141. When rotating simultaneously, the storage disk 131 and the accommodating disk 141 drive the aerosol generating substrate 12 to be heated to be fed, and drive the atomized aerosol generating substrate 12 to be wound around the accommodating disk 141. In some other embodiments, an accommodating disk 141 is not limited to a driving wheel, and may alternatively include a driven wheel. A storage disk 131 is not limited to a driven wheel, and may alternatively include a driving wheel. In some embodiments, a storage disk 131 and an accommodating disk 141 may alternatively be rotatably arranged separately. That is, the storage disk 131 may be driven to rotate by one set of driving structures, and the accommodating disk 141 may be driven to rotate by another set of driving structures.

In the embodiment, the size of accommodating space of the second accommodating structure 14 may be greater than the size of accommodating space of the first accommodating structure 13. Specifically, the radial dimensions of the third disk body 1411 and the fourth disk body 1412 are greater than the radial dimension of the first disk body 1311, and the radial dimensions of the third disk body 1411 and the fourth disk body 1412 are greater than the radial dimension of the second disk body 1312, such that the size of accommodating space formed between the third disk body 1411 and the fourth disk body 1412 is greater than the size of accommodating space formed between the second disk body 1312 and the first disk body 1311. Clearly, understandably, in some other embodiments, the cross-sectional size of a winding drum may be set less than the cross-sectional size of a reel, such that the size of accommodating space formed between a third disk body 1411 and a fourth disk body 1412 is greater than the size of accommodating space formed between a second disk body 1312 and a first disk body 1311. In some other embodiments, the size of accommodating space of a second accommodating structure 14 may alternatively be equal to or less than the size of accommodating space of a first accommodating structure 13.

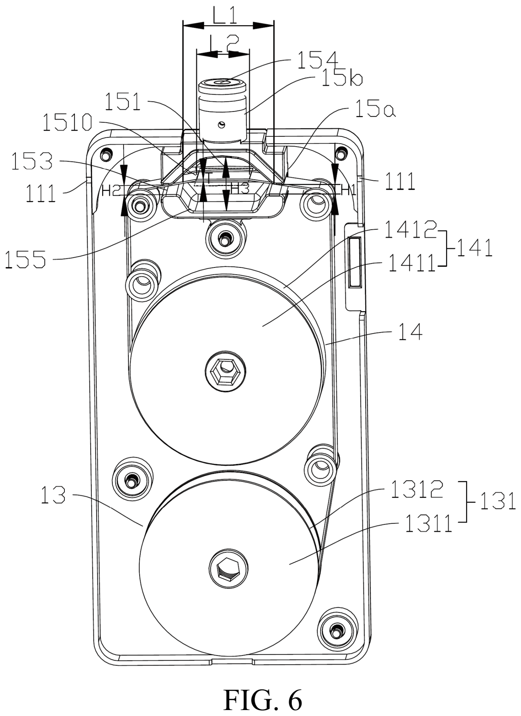

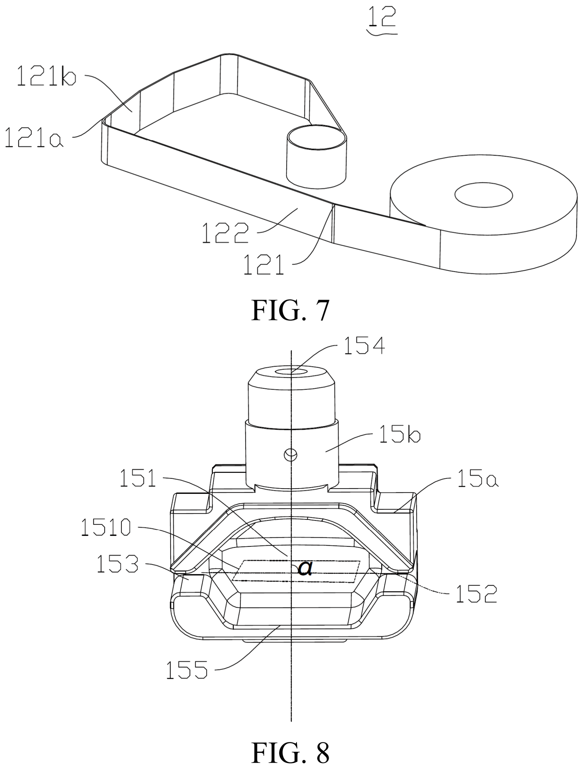

As shown in FIG. 6 and FIG. 8, in the embodiment, the aerosol generating substrate assembly 10 further includes an atomization housing 15. The atomization housing 15 is partially arranged on a box body 11. The atomization housing 15 includes a body portion 15a and an extension portion 15b arranged on the body portion 15a. The body portion 15a may be embedded into the box body 11.

An atomization cavity 151 is formed on an inner side of the atomization housing 15, and the atomization cavity 151 has an inlet 152 and an outlet 153. The inlet 152 and the outlet 153 are provided on the atomization housing 15. The atomization cavity 151 is formed on an inner side of the body portion 15a, and the atomization cavity 151 is defined by space in which a heating structure 22 heats an aerosol generating substrate 12. The inlet 152 is provided on one side of the body portion 15a, is in communication with the atomization cavity 151, and is used for allowing the aerosol generating substrate 12 to be heated to enter the atomization cavity 151. In some embodiments, an inlet 152 may be provided opposite one of air intake vents 111, and may further be correspondingly in communication with the air intake vent. The outlet 153 is provided on the other side of the body portion 15a, is in communication with the atomization cavity 151, and is used for allowing the atomized aerosol generating substrate 12 to be conveyed out. In some embodiments, an outlet 153 may be provided opposite another air intake vent 111, and may further be correspondingly in communication with the air intake vent. Understandably, in some other embodiments, only an inlet 152 or an outlet 153 may alternatively be connected to an air intake vent 111. The inlet 152 and the outlet 153 are provided opposite each other, and are located in or close to a plane in which a center of the atomization cavity 151 is located. The extension portion 15b is arranged on the body portion 15a, may pass out from the box body 11, and is used for connecting to a mouthpiece assembly 30. The extension portion 15b is cylindrical, and is of a two-end through structure, and an air outlet channel 154 in communication with the atomization cavity 151 is formed on an inner side of the extension portion, and is used for allowing an aerosol formed by atomization to be output. The inlet 152 and the outlet 153 may be provided on two different sides of the air outlet channel 154, and are provided on two opposite sides of the air outlet channel 154, for example. Clearly, understandably, in some other embodiments, an inlet 152 and an outlet 153 may alternatively be provided on two adjacent sides of an air outlet channel 154. In some other embodiments, an inlet 152 and an outlet 153 may further be provided on the same side of an air outlet channel 154. In some embodiments, an atomization cavity 151 has an opening 155, and the opening 155 may be provided on one side of a body portion 15a arranged opposite a heating structure 22, and is used for allowing the heating structure 22 to be inserted into the atomization cavity 151 from the opening.

In the embodiment, the atomization cavity 151 may be irregular. Clearly, understandably, in some other embodiments, an atomization cavity 151 may alternatively be regular, such as cubic, conical, cylindrical or other shapes. The height H3 of the atomization cavity 151 may range from 6.0 mm to 7.0 mm (values at two ends are included). Specifically, in some embodiments, the height H3 of the atomization cavity 151 may be selected as 6.7 mm. Clearly, understandably, in some other embodiments, the height H3 of an atomization cavity 151 may be not limited to 6.7 mm. It should be noted that a direction in which the height H3 of the atomization cavity 151 is located may be the same as a direction in which the thickness of the aerosol generating substrate 12 in the atomization cavity 151 is located. That is, the direction is perpendicular to a movement direction of the aerosol generating substrate 12.

In the embodiment, a heating region 1510 is formed in the atomization cavity 151. The heating region 1510 is a region in which the heating structure 22 is located, i.e. a region in which the aerosol generating substrate 12 is heated and atomized. The aerosol generating substrate 12 is movably arranged in the heating region 1510 in a penetrating manner and heated when passing through the heating region 1510. In some embodiments, a heating region 1510 has a start point and an end point in a direction in which an aerosol generating substrate 12 is arranged in an atomization cavity 15 in a penetrating manner. In the embodiment, a start point and an end point may be located on two opposite sides of the heating region 1510, the start point may be arranged towards the inlet 152, and the end point may be arranged towards the outlet 153. The aerosol generating substrate 12 may move from the start point to the end point sequentially, and further may pass through the heating region 1510. In some embodiments, the width W of an atomization cavity 151 is greater than or equal to the width of a heating region 1510, and the width of the heating region 1510 is greater than the width w of an aerosol generating substrate 12, thereby ensuring that the aerosol generating substrate 12 may be sufficiently heated in the heating region 1510. In some embodiments, the length L1 of an atomization cavity 151 is greater than or equal to the length L2 of a heating region 1510. That is, the length of the atomization cavity 151 is greater than a connection line between a start point and an end point of the heating region 1510, such that heating of an aerosol generating substrate 12 is concentrated in the atomization cavity 151, it is ensured that an aerosol generated by heating is accumulated in the atomization cavity 151, and overflow of the aerosol is avoided. L1 may be 10 mm, and L2 may be 9 mm.

In the embodiment, the ratio of the height H1 of the inlet 152 to the height H3 of the atomization cavity 151 is 0.12 to 0.40, and the height H1 of the inlet 152 is greater than the thickness T of the aerosol generating substrate 12, such that the aerosol generating substrate 12 enters the inlet. By configuring the ratio of the height H1 of the inlet 152 to the height H3 of the atomization cavity 151 to be 0.12 to 0.40, the situation that the size of the inlet 152 is excessively small, such that a pressure of the atomization cavity 151 is excessively small to cause inhaling turbulence may be prevented, and inhaling experience is prevented from being influenced. Moreover, the situation that the size of the inlet 152 is excessively large, such that an aerosol generated by heating the aerosol generating substrate 12 in the atomization cavity 151 overflows from the inlet 152 may be prevented. That is, the aerosol is prevented from leaking. It should be noted that a direction in which the height H1 of the inlet 152 is located may be the same as a direction of the thickness T of the aerosol generating substrate 12. That is, the direction may be perpendicular to a direction in which the aerosol generating substrate 12 moves towards the atomization cavity 151. In some embodiments, the height H1 of an inlet 152 may range from 0.8 mm to 2.5 mm (values at two ends are included). Specifically, the height H1 of the inlet 152 may be selected as 1.2 mm.

In the embodiment, the ratio of the height H2 of the outlet 153 to the height H3 of the atomization cavity 151 is 0.12 to 0.40, and the height of the outlet is greater than the thickness T of the aerosol generating substrate 12, such that the aerosol generating substrate 12 is output. By configuring the ratio of the height H2 of the outlet 153 to the height H3 of the atomization cavity 151 to be 0.12 to 0.40, the situation that the size of the outlet 153 is excessively small, such that a pressure of the atomization cavity 151 is excessively small to cause inhaling turbulence may be prevented, and inhaling experience is prevented from being influenced. Moreover, the situation that the size of the outlet 153 is excessively large, such that an aerosol generated by heating the aerosol generating substrate 12 in the atomization cavity 151 overflows from the outlet 153 may be prevented. That is, the aerosol is prevented from leaking. It should be noted that a direction in which the height H2 of the outlet 153 is located may be the same as a direction of the thickness of the aerosol generating substrate 12. That is, the direction may be perpendicular to a direction in which the aerosol generating substrate 12 moves towards the atomization cavity 151. In some embodiments, the height H2 of an outlet 153 may range from 0.8 mm to 2.5 mm (values at two ends are included). Specifically, the height of the outlet 153 may be equal to the height H1 of an inlet 152. In some embodiments, the height H2 of an outlet 153 may be selected as 1.2 mm.

In the embodiment, the size of the inlet 152 and the size of the outlet 153 depend on the width of the aerosol generating substrate 12. In some embodiments, the width W of an inlet 152 may be set greater than the width w of an aerosol generating substrate 12, such that the aerosol generating substrate 12 enters an atomization cavity 151 from the inlet 152. In some embodiments, the width W of an outlet 153 may be set greater than the width w of an aerosol generating substrate 12, such that the aerosol generating substrate 12 is output from the outlet 153.

In the embodiment, the air outlet channel 154 may be longitudinally arranged in a direction perpendicular to a movement direction of the aerosol generating substrate 12, and is cylindrical or conical. In some embodiments, an air outlet channel 154 may alternatively be formed only by providing an air outlet on an atomization housing 15. The air outlet channel 154 has an axial direction, and the axial direction is a direction parallel to an extension direction of a central axis of the air outlet channel 154. In the embodiment, the axial direction is the same as the extension direction of the air outlet channel 154. An aerosol generated by heating the aerosol generating substrate 12 may be output to the outside through the axial direction of the air outlet channel 154. The axial direction is not parallel to the movement direction of the aerosol generating substrate 12 in the atomization cavity 151. That is, a set included angle α may be formed between the axial direction of the air outlet channel and the movement direction of the aerosol generating substrate 12 in the atomization cavity 151. The set included angle α is greater than zero degree and less than one hundred and eighty degrees. Thus, output efficiency of the aerosol can be improved, and an output effect of the aerosol can be ensured.

Further, the set included angle α is formed between the axial direction of the air outlet channel 154 and a penetration direction in which the aerosol generating substrate 12 is arranged in the heating region in a penetrating manner. Specifically, the set included angle α is formed between connecting lines between a central axis of the air outlet channel 154 and the start point of the heating region 1510 and between the central axis of the air outlet channel and the end point. In some embodiments, a set included angle α may be selected as 90 degrees. That is, the air outlet channel 154 may be arranged in an axial direction of the heating region 1510, and an axis of the air outlet channel 154 may coincide with the axial direction of the heating region 1510 such that it can be ensured that the air outlet channel 154 is arranged in a direction in which the aerosol is output when the aerosol generating substrate 12 is heated in the heating region 1510, the aerosol generated by the aerosol generating substrate 12 can be rapidly and massively output from the air outlet channel 154, an output speed of the aerosol of the present disclosure is increased, taste of the output aerosol is ensured, and user experience is further improved.

As further shown in FIG. 5 and FIG. 6, in the embodiment, the aerosol generating substrate assembly 10 further includes a guide structure 16. The guide structure 16 is arranged in the box body 11 and is used for guiding transmission on the aerosol generating substrate 12. In some embodiments, a guide structure 16 includes a first guide roller 161, a second guide roller 162, a third guide roller 163, and a fourth guide roller 164 which are sequentially arranged. The first guide roller 161 is arranged on one side of the first accommodating structure 13, and is used for guiding a progress of the aerosol generating substrate 12 output from the first accommodating structure 13. The second guide roller 162 and the third guide roller 163 are arranged on two opposite sides of an atomization housing 15 respectively. The second guide roller 162 is located on one side of the inlet 152 away from the outlet 153, and the third guide roller 163 is located on one side of the outlet 153 away from the inlet 152. The aerosol generating substrate 12 may be better attached to the heating structure 22 through the second guide roller 161 and the third guide roller 163. The fourth guide roller 164 is located on one side of the second accommodating structure 14. A connecting line between the fourth guide roller 164 and the third guide roller 163 and a connecting line between the second guide roller 162 and the third guide roller 163 are arranged at an included angle, and the included angle is an angle greater than 0 degree and less than 180 degrees. When the storage disk 131 and the accommodating disk 141 rotate, the aerosol generating substrate 12 to be heated may enter the atomization cavity 151 along the first guide roller 161, the second guide roller 162, and the inlet 152. After atomization, the storage disk 131 and the accommodating disk 141 continue rotating, and the atomized aerosol generating substrate 12 may be output from the outlet 153 and sequentially accommodated into the accommodating disk 141 through the third guide roller 163. In some embodiments, a guide structure 16 is not limited to including four guide rollers, or the wire structure 16 may include three guide rollers. In some embodiments, a guide structure 16 is also not limited to a roller as long as the guide structure may guide movement of an aerosol generating substrate 12.

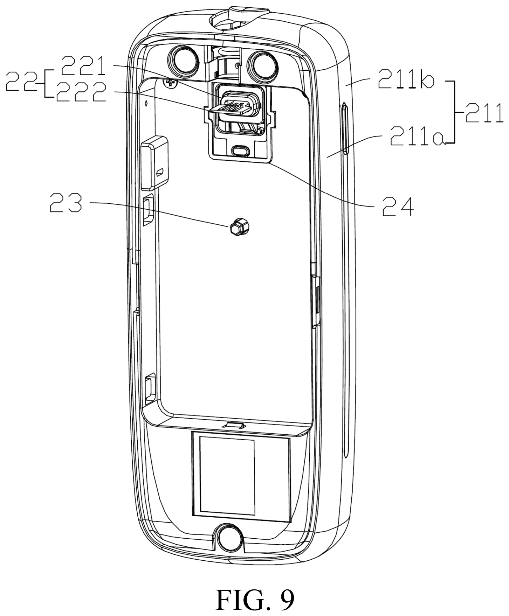

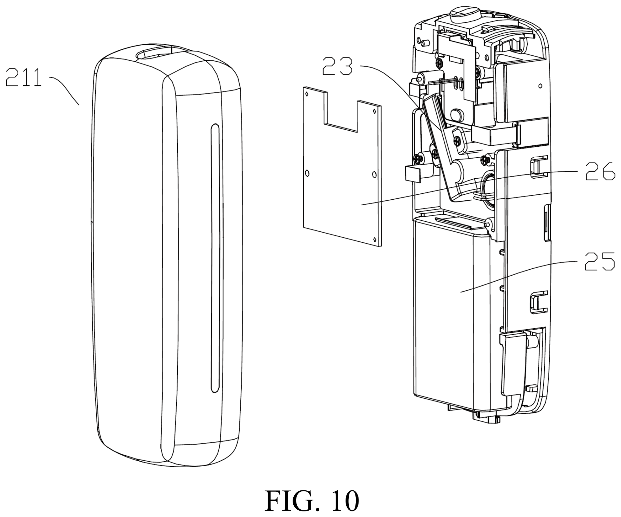

With reference to FIG. 1 to FIG. 3, FIG. 9 and FIG. 10 together, the aerosol generating device 20 includes a machine body 21 and a heating structure 22. The machine body 21 may be used for accommodating an aerosol generating substrate assembly 10. The heating structure 22 is mounted on the machine body 21, may be inserted into the aerosol generating substrate assembly 10, and is used for heating an aerosol generating substrate 12 in the aerosol generating substrate assembly 10 to generate an aerosol.

In the embodiment, the machine body 21 includes an enclosure 211 and a cover body 212. The enclosure 211 includes a first housing 211a and a second housing 211b. The first housing 211a is arranged on the second housing 211b, an accommodating cavity 2111 is provided on the first housing 211a, and the accommodating cavity 2111 is used for accommodating the aerosol generating substrate assembly 10. An assembly port 2112 is provided on a side wall of the first housing 211a, and the assembly port 2112 is used for allowing the aerosol generating substrate 12 to be loaded into the accommodating cavity 2111. The cover body 212 is arranged at the assembly port 2112, and detachably covers the accommodating cavity 2111. By opening the cover body 212, the aerosol generating substrate assembly 10 may be convenient to remove and replace.

In the embodiment, the heating structure 22 is mounted on the first housing 211a, is located on a bottom wall of the accommodating cavity 2111, and protrudes towards the accommodating cavity 2111. When the aerosol generating substrate assembly 10 is mounted in the accommodating cavity 2111, the heating structure 22 may be inserted into an atomization housing 15 of the aerosol generating substrate assembly 10, and is used for heating the aerosol generating substrate 12 to be heated that enters the atomization cavity 151. In some embodiments, a heating structure 22 includes a base 221 and a heating element 222. The base 221 is mounted on a bottom wall of an accommodating cavity 2111, and the heating element 222 is mounted on the base 221. In some embodiments, the heating element 222 includes a metal heating element. Clearly, understandably, in some other embodiments, a heating element 222 may further include a ceramic heating element and a glass heating element. The heating element 222 may be sheet-like, filamentous, or cylindrical. In some other embodiments, a heating structure 22 may alternatively be an electromagnetic heating structure, and may not be limited to being inserted into an atomization housing 15.

In the embodiment, the aerosol generating device 20 further includes a driving assembly 23. The driving assembly 23 may be mounted on the machine body 21. Specifically, the driving assembly 23 is mounted between the first housing 211a and the second housing 211b, and the part of the driving assembly may extend into the accommodating cavity 2111 to be connected to the second accommodating structure 14 or the first accommodating structure 13, and is used for transferring the aerosol generating substrate 12 in the aerosol generating substrate assembly 10 to the heating structure 22. In some embodiments, a driving assembly 23 may be an electric driving structure. For example, the driving assembly 23 may be an electric motor or a reduction gearbox. An output shaft of the driving assembly 23 may penetrate the accommodating cavity 2111 from one side of the first housing the first housing 211a arranged opposite to the accommodating cavity 2111, and is connected to the second accommodating structure 14 or the first accommodating structure 13. That is, the output shaft may be connected to the storage disk 131 or the accommodating disk 141, and is used for driving the storage disk 131 and a second accommodating wheel 132 to rotate. Clearly, understandably, in some other embodiments, a driving assembly 23 may alternatively be not limited to an electric driving structure, and may be a manual driving structure, such as a handle or a hand wheel.

In the embodiment, the aerosol generating device 20 further includes a sealing structure 24. The sealing structure 24 is arranged in the accommodating cavity 2111 and is located at a periphery of the heating structure 22. Specifically, the sealing structure 24 may be a sealing ring. When the aerosol generating substrate assembly 10 is loaded into the accommodating cavity 2111, the sealing structure 24 may sealing a gap between an end surface on which an opening 155 of the atomization cavity 151 of the aerosol generating substrate assembly 10 is located and a bottom wall of the accommodating cavity 2111 to prevent the aerosol from leaking.

In the embodiment, the aerosol generating device 20 further includes a power supply 25. The power supply 25 is arranged in the enclosure 211, and is specifically located between the first housing 211a and the second housing 211b. The power supply 25 is used for supplying power to the driving assembly 23 and the heating structure 22. In some embodiments, a power supply 25 may be a battery or a chemical reactant.

In the embodiment, the aerosol generating device 20 further includes a main control board 26. The main control board 26 is arranged in the enclosure 211, and is specifically located between the first housing 211a and the second housing 211b. The main control board 26 is connected to the power supply 25, the driving assembly 23, and the heating structure 22.

In the embodiment, the aerosol generating system 100 further includes a mouthpiece assembly 30. The mouthpiece assembly 30 may be assembled on the atomization housing 15. Specifically, the mouthpiece assembly 30 may sleeve the extension portion 15b, and a user allows a user to inhale the aerosol output from the atomization cavity 151. In some embodiments, a mouthpiece assembly 30 may be approximately cylindrical. Clearly, understandably, in some other embodiments, a mouthpiece assembly 30 is not limited to being cylindrical, and may alternatively be flat cylindrical or other shapes.

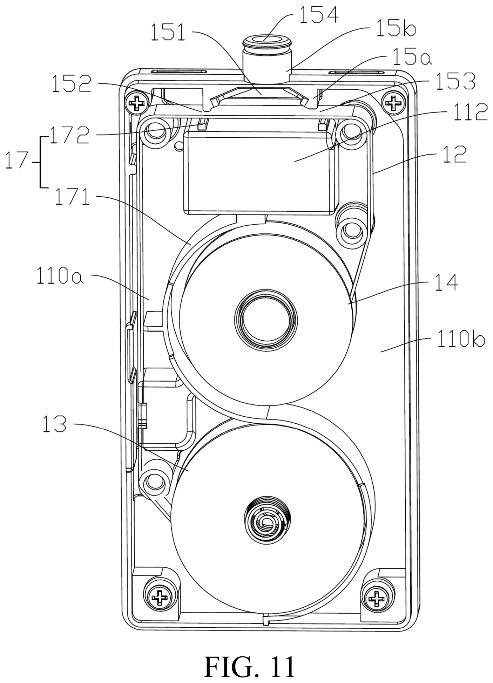

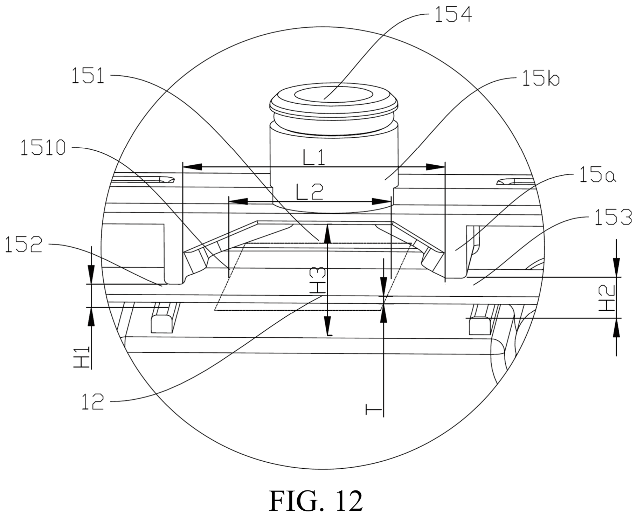

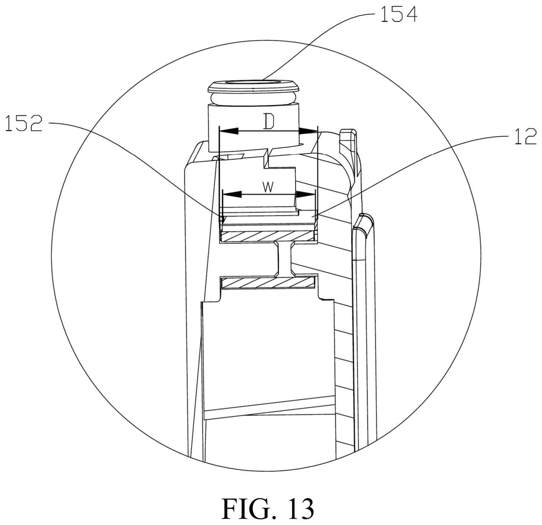

FIG. 11 to FIG. 13 show Embodiment 2 of an aerosol generating system 100 of the present disclosure. The embodiment differs from Embodiment 1 in that in the embodiment, a first box body 11a protrudes inwards to be provided with an accommodating portion 112, one side of the accommodating portion 112 arranged opposite to a second box body 11b may define an accommodating groove, and the accommodating groove may be used for accommodating a heating structure 22.

In the embodiment, an atomization housing 15 may be integrally formed on a box body 11. That is, the atomization housing may be integrally formed on the first box body 11a or the second box body 11b. A body portion 15a is formed in the first box body 11a, and may be of a gradually narrowed structure towards an extension portion 15b, and one end of the body portion away from the extension portion 15b is opened. The body portion 15a and the accommodating portion 112 are arranged opposite each other, and are arranged at an interval, and a channel for allowing an aerosol generating substrate 12 to move is formed in a gap between the body portion and the accommodating portion. The body portion 15a may match the accommodating portion 112 to define an atomization cavity 151, an inlet 152, and an outlet 153. The inlet 152 and the outlet 153 are located on two opposite sides of the atomization cavity 151. In the embodiment, the height H2 of the outlet 153 may be greater than the height H1 of the inlet 152. Optionally, the height H2 of the outlet 153 may be 1.1 mm, and the height H1 of the inlet 152 may be 0.8 mm. The height H2 of the outlet 153 may be set greater than the height H1 of the inlet 152 because a medium layer 122 is separated from a base band 121 or expands and hardens after the aerosol generating substrate 12 is heated. Thus, the heated aerosol generating substrate 12 may be conveniently output by setting the height H2 of the outlet 153 to be greater than the height H1 of the inlet 152. In the embodiment, the width D of the inlet 152 and the outlet 153 is greater than the width w of the aerosol generating substrate 12. In some embodiments, optionally, the width D of the inlet 152 and the outlet 153 may be selected as 7.5 mm. The width w of the aerosol generating substrate 12 may be selected as 5 mm.

In the embodiment, storage space 110a and accommodating space 110b are formed in the box body 11. Specifically, the storage space 110a may be formed in space in which a first accommodating structure 13 is located, and the accommodating space may be formed in space in which a second accommodating structure 14 is located. In the embodiment, at least one blocking structure 17 is arranged between the storage space 110a, the accommodating space 110b, and the atomization cavity 151. Specifically, in the embodiment, the at least one blocking structure 17 may include a first blocking structure 171. The first blocking structure 171 is arranged between the storage space 110a and the accommodating space 110b. The first blocking structure 171 may be an S-shaped retaining wall, and may separate the storage space 110a and the accommodating space 110b. The retaining wall may be integrally formed on the first box body 11a or the second box body 11b, and makes close contact with the first box body 11a and the second box body 11b to prevent residues and smell after the medium layer 122 in the accommodating space 110b is atomized from entering the storage space 110a. The at least one blocking structure 17 may further include at least one second blocking structure 172. The at least one second blocking structure 172 may be arranged between the storage space 110a and the atomization cavity 151, and may be used for preventing the aerosol in the atomization cavity 151 from overflowing towards the storage space 110a. The second blocking structure 172 may be a blocking rib arranged on one side of the inlet 152 close to the storage space 110a, or a narrowed structure formed by narrowing the inlet 152. In some embodiments, the blocking structure 17 may further be a cap covering the first accommodating structure 13 and/or the second accommodating structure 14. The cap may be integrally formed in the first box body 11a or the second box body 11b to make close contact with the first box body 11a or the second box body 11b.

Understandably, the above embodiments only describe the implementations of the present disclosure specifically and in detail, but cannot be construed as a limitation on the scope of patent of the present disclosure accordingly. It should be pointed out that for those of ordinary skill in the art, without departing from the concept of the present disclosure, the above technical features can be freely combined, and several deformations and improvements can be further made, all of which fall within the scope of protection of the present disclosure. Thus, any equivalent transformations and modifications made to the scope of the claims of the present disclosure shall fall within the scope of the claims of the present disclosure.

## Claims

1. An aerosol generating substrate assembly, comprising: a banded aerosol generating substrate (12) arranged movably and an atomization cavity (151) for allowing the aerosol generating substrate (12) to be heated and atomized to form an aerosol, wherein
the atomization cavity (151) is provided with an air outlet channel (154), the air outlet channel (154) has an axial direction, and a set included angle is formed between the axial direction of the air outlet channel and a movement direction of the aerosol generating substrate (12) in the atomization cavity (151); and the set included angle is greater than zero degree and less than one hundred and eighty degrees.

2. The aerosol generating substrate assembly of claim 1, wherein a heating region is arranged in the atomization cavity (151), and the aerosol generating substrate (12) is movably arranged in the heating region in a penetrating manner; and
the set included angle is formed between the axial direction of the air outlet channel (154) and a penetration direction in which the aerosol generating substrate (12) is arranged in the heating region in a penetrating manner.

3. The aerosol generating substrate assembly of claim 2, wherein the heating region has a start point and an end point in the direction in which the aerosol generating substrate (12) is arranged in a penetrating manner; and the set included angle is formed between connecting lines between a central axis of the air outlet channel (154) and the start point and between the central axis of the air outlet channel and the end point.

4. The aerosol generating substrate assembly of claim 1, wherein the atomization cavity (151) has an inlet (152) and an outlet (153); the inlet (152) is in communication with the atomization cavity (151) for the aerosol generating substrate (12) to be heated to enter the atomization cavity (151); and the outlet (153) is in communication with the atomization cavity (151) for the heated aerosol generating substrate (12) to output.

5. The aerosol generating substrate assembly of claim 4, wherein the inlet (152) and the outlet (153) are provided on two different sides of the air outlet channel (154); or
the inlet (152) and the outlet (153) are provided on the same side of the air outlet channel (154).

6. The aerosol generating substrate assembly of claim 4, wherein the height of the outlet (153) is greater than or equal to the height of the inlet (152); and
the height of the inlet (152) is greater than or equal to the thickness of the aerosol generating substrate (12).

7. The aerosol generating substrate assembly of claim 6, wherein the heights/height of the inlet (152) and/or the outlet (153) range/ranges from 0.8 mm to 2.5 mm; and/or
the thickness of the aerosol generating substrate (12) ranges from 0.15 mm to 0.35 mm.

8. The aerosol generating substrate assembly of claim 5, wherein the widths/width of the inlet (152) and/or the outlet (153) are/is greater than the width of the aerosol generating substrate (12).

9. The aerosol generating substrate assembly of claim 5, wherein the ratios of the heights/height of the inlet (152) and/or the outlet (153) to the height of the atomization cavity (151) are/is 0.12 to 0.40.

10. The aerosol generating substrate assembly of claim 2, wherein the width of the atomization cavity is greater than or equal to the width of the heating region; and
the width of the heating region is greater than the width of the aerosol generating substrate (12).

11. An aerosol generating system, comprising: an aerosol generating device (20) and the aerosol generating substrate assembly (10) of any one of claims 1 to 10.
